# EUROPÄISCHE PATENTSCHRIFT

(11) **EP 3 397 202 B1**
(45) Veröffentlichungstag und Bekanntmachung des Hinweises auf die Patenterteilung: **29.01.2020**
(21) Anmeldenummer: 16820301.6
(22) Anmeldetag: 29.12.2016
(51) Int. Cl.: A61F 2/07, A61F 2/06

(54) **SELBSTEXPANDIERENDE GEFÄSSPROTHESE**
SELF-EXPANDING VASCULAR PROSTHESIS
PROTHÈSE VASCULAIRE AUTOEXPANSIBLE

(30) Priorität: 30.12.2015 DE 102015123000
(43) Veröffentlichungstag der Anmeldung: 07.11.2018
(73) Patentinhaber: JOTEC GmbH, 72379 Hechingen (DE)
(72) Erfinder: SZOPINSKI, Piotr, 03604 Warschau (PL); WOERNE, Christian, 73760 Ostfildern (DE); WALTHER, Michael, 24632 Lentföhrden (DE)
(74) Vertreter: Witte, Weller & Partner Patentanwälte mbB
(86) Internationale Anmeldenummer: PCT/EP2016/082798
(87) Internationale Veröffentlichungsnummer: WO 2017/114879

(56) Entgegenhaltungen:
- EP-A2- 2 777 610
- WO-A1-2010/024879
- WO-A1-2011/047004
- WO-A1-2015/061669
- WO-A1-2015/109375
- DE-A1-102012 103 986

## Beschreibung

Die vorliegende Erfindung betrifft eine selbstexpandierende Gefäßprothese zur Implantation in ein Blutgefäß eines Patienten, mit einem einen hohlzylindrischen Grundkörper, sowie eine erste und eine zweite Öffnung aufweisenden Gefäßprothesenrumpf und mit mindestens zwei vom Gefäßprothesenrumpf abgehenden Gefäßprothesen-Seitenästen.

Allgemein ist es bekannt, intraluminale Gefäßprothesen oder - implantate, die auch als endovaskuläre Gefäßstents/-stentgrafts bezeichnet werden, zur Behandlung von geschwächten, verletzten, gerissenen oder aneurysmatischen Gefäßen einzusetzen. Hierfür wird an der erkrankten oder verletzten Stelle des Gefäßes ein Gefäßimplantat bzw. Stentgraft freigesetzt, der die Funktionalität des ursprünglichen Gefäßes wieder herstellt bzw. die noch bestehende Gefäßintegrität unterstützt.

Dabei versteht man unter einem Aneurysma eine Ausweitung oder Aussackung eines arteriellen Blutgefäßes infolge angeborener oder erworbener Wandveränderungen. Die Aussackung kann dabei die Gefäßwand als Ganzes erfassen, oder, wie bei dem sogenannten falschen Aneurysma bzw. der sogenannten Dissektion, es tritt Blut aus dem Lumen des Gefäßes zwischen die Gefäßwandschichten und schert diese auseinander. Die Nichtbehandlung eines Aneurysma kann im fortgeschrittenen Stadium zu einer Ruptur der Arterie führen, mit der Folge, dass der Patient innerlich verblutet. Ursache eines thorakalen und thorakoabdominalen Aortenaneurysmas können Arteriosklerose, Bluthochdruck sowie Entzündungsprozesse der Gefäßwand sein. Auch Verletzungen des Brustkorbes durch schwere Unfälle können akut oder chronisch zu einem Aortenaneurysma führen.

Die zur Behandlung von Aneurysmen verwendeten selbstexpandierenden Gefäßprothesen bestehen im Allgemeinen aus einem hohlzylindrischen Metallrahmen bzw.-gerüst, dessen Mantelfläche mit einer Textil- oder Polymerfolie abgedeckt ist, so dass sich ein hohlzylindrischer Körper ergibt. Zur Implantation wird das Gefäßimplantat radial zusammengedrückt, so dass sich seine Querschnittsfläche deutlich verringert. Das Gefäßimplantat wird dann mit Hilfe eines Einführsystems in den Bereich des Aneurysma gebracht, wo es freigesetzt wird. Auf Grund der Federwirkung des Metallrahmens/-gerüsts expandiert das Gefäßimplantat wieder in seine ursprüngliche Form und spannt dabei seine Mantelfläche auf, die sich proximal und distal von dem Aneurysma innen in dem Blutgefäß verklemmt. Auf diese Weise fließt das Blut jetzt durch das Gefäßimplantat und eine weitere Belastung der Aussackung wird verhindert.

Der Metallrahmen solcher Gefäßimplantate besteht in der Regel bspw. aus einem Drahtgeflecht oder aus hintereinander angeordneten, mäanderförmig umlaufenden, sogenannten Stentfedern, die ggf. über Verbindungsstützen aus Draht miteinander verbunden sind, oder die lediglich über das Implantatmaterial miteinander verbunden sind. Das Drahtgeflecht bzw. die Stentfedern sind üblicherweise aus einem Shape-Memory-Material, in der Regel aus Nitinol, wodurch die Stentfedern nach Einbringung in ein Gefäß zur Freisetzung wieder in den expandierten Zustand übergehen und das Gefäßimplantat dadurch "aufspannen".

Aneurysmen treten häufig im Bereich der Baucharterie (Aorta abdominalis) oder Brustarterie (Aorta thoracica) auf, wobei ein thorakales Aneurysma im sogenannten aufsteigenden Ast der Aorta (Aorta ascendens), im Aortenbogen und/oder im absteigenden Ast der Aorta auftreten kann.

Beim thorakoabdominellen Aortenaneurysma bleibt das Aneurysma nicht nur auf einen begrenzten Teil der Hauptschlagader begrenzt, sondern findet sich sowohl im Brustraum, also im Thorax als auch im Bauchraum (Abdomen).

Das thorakoabdominelle Aortenaneurysma ist ein sehr komplexex Krankheitsbild und schwierig zu behandeln. Vor der Etablierung von Aortenstents war es nahezu immer notwendig, sowohl Brustkorb als auch Bauchraum zu öffnen. Heute kann alternativ, im Rahmen von Hybrid-Operationen, auch eine Eröffnung der Bauchhöhle erfolgen in Kombination mit einem Aortenstent, bzw. eine totale endovaskuläre Behandlung von thorakoabdominellen Aortenaneurysmen.

Erschwerend kommt beim thorakoabdominellen Aortenaneurysma meist immer hinzu, dass sämtliche wichtigen Organarterien (Darmarterien, Truncus coeliacus, Nierenarterien) bei der Reparation mit betroffen sind und in irgendeiner Form rekonstruiert werden müssen. Daher ist die Therapie des thorakoabdominellen Aneurysmas schwierig, mit Komplikationen behaftet, und wird regelmäßig von speziellen Zentren durchgeführt.

Die bei der Therapie eingesetzten Gefäßprothesen müssen dabei viele Erfordernisse erfüllen, insbesondere im Hinblick auf die Größe, Länge und Morphologie des zu überbrückenden Aneurysmas und der nativen Aorta; ferner spielt die Durchlässigkeit der Segmentarterien in diesem Bereich, die Koexistenz weiterer Aneurysmen und der Durchmesser der Iliacal- und Femoralgefäße eine große Rolle.

Aus der EP 2 777 610 A2 ist eine Gefäßprothese bekannt, die einen hohlzylindrischen Hauptkörper sowie einen Verlängerungskörper aufweist, der innerhalb des Grundkörpers freigesetzt werden kann.

Nach wie vor besteht daher ein großes Bedürfnis nach Stent-/Stentgraftsystemen, bzw Gefäßprothesen, mit Hilfe derer der oben geschilderte Eingriff vereinfacht und zeitlich verkürzt werden könnte.

Aufgabe der vorliegenden Erfindung ist es daher, ein System bereitzustellen, mit welchem thorakoabdominelle Aneurysmen schnell und unkompliziert behandelt werden können.

Erfindungsgemäß wird diese Aufgabe gelöst durch eine selbstexpandierende Gefäßprothese zur Implantation in ein Blutgefäß eines Patienten, insbesondere in den thorakoabdominellen Aorten-Bereich, mit einem einen hohlzylindrischen Grundkörper, sowie mit einem eine erste und eine zweite Öffnung aufweisenden Gefäßprothesenrumpf und mit mindestens zwei vom Gefäßprothesenrumpf abgehenden Gefäßprothesen-Seitenästen, die einstückig mit dem Gefäßprothesenrumpf ausgebildet sind, wobei der Gefäßprothesenrumpf einen ersten Gefäßprothesenrumpf-Längsabschnitt, der die erste Öffnung umfasst, einen zweiten Gefäßprothesenrumpf-Längsabschnitt, der die zweite Öffnung umfasst, und zumindest zwei zwischen dem ersten und dem zweiten Gefäßprothesenrumpf-Längsabschnitt angeordneten mittlere Gefäßprothesenrumpf-Längsabschnitte aufweist, die hintereinander angeordnet sind, und wobei die mindestens zwei vom Gefäßprothesenrumpf abgehenden Gefäßprothesen-Seitenäste in den mittleren Gefäßprothesenrumpf-Längsabschnitten angeordnet sind, wobei ferner der erste und der zweite Gefäßprothesenrumpf-Längsabschnitt jeweils einen konstanten Durchmesser über ihre jeweilige Längsrichtung aufweisen und in Bezug aufeinander konzentrisch angeordnet sind und wobei der Durchmesser des ersten Gefäßprothesenrumpf-Längsabschnitts größer ist als der Durchmesser des zweiten Gefäßprothesenrumpf-Längsabschnitts; die erfindungsgemäße Gefäßprothese zeichnet sich ferner dadurch aus, dass sich der erste und der zweite mittlere Gefäßprothesenrumpf-Längsabschnitt jeweils zum zweiten Gefäßprothesenrumpf-Längsabschnitt hin nur in jeweils einem Teil des Umfangs und nicht über den gesamtem jeweiligen Umfang, verjüngen, wobei die sich jeweils verjüngenden Umfangsteile des ersten und des zweiten mittleren Gefäßprothesenrumpf-Längsabschnitts in Umfangsrichtung versetzt zueinander angeordnet sind, und dass zumindest ein Gefäßprothesen-Seitenast im ersten und zumindest ein zweiter Gefäßprothesen-Seitenäste im zweiten mittleren Gefäßprothesenrumpf-Längsabschnitt in dem sich jeweils verjüngenden Umfangsteil angeordnet sind.

Die der Erfindung zugrunde liegende Aufgabe wird ferner durch die Verwendung der erfindungsgemäßen selbstexpandierenden Gefäßprothese zur Behandlung eines thorakalen-abdominalen Aneurysmas gelöst.

Die der Erfindung zugrunde liegende Aufgabe wird dadurch vollkommen gelöst.

Mit der erfindungsgemäßen selbstexpandierenden Gefäßprothese wird ein Gefäßprothesen-Modul bereitgestellt, mit dem thorakoabdominelle Aneurysmen erfolgreich überbrückt werden können, wobei gleichzeitig die Versorgung der abgehenden Seitengefäße, also bspw. der Viszeralarterien, durch die Gefäßprothesen-Seitenäste sicher gewährleistet wird. Die vom Gefäßprothesenrumpf abgehenden Gefäßprothesen-Seitenästen, die einstückig mit dem Gefäßprothesenrumpf ausgebildet sind, erstrecken sich dabei in distale Richtung und parallel zur Längsrichtung des Gefäßprothesenrumpfes, und sind - zusammen mit dem Gefäßprothesenrumpf - in demselben Gefäß freisetzbar; dies bedeutet, dass die Seitenäste und die Öffnungen des Gefäßprothesenrumpfes in gleichen Hauptgefäß freigesetzt werden bzw. freisetzbar sind. Erfindungsgemäß sind die Seitenäste der erfindungsgemäßen Gefäßprothese daher, insbesondere in Bezug auf ihre Länge und einstückige Ausbildung am Gefäßprothesenrumpf, so dimensioniert und ausgestaltet, dass diese im das Aneurysma tragende Hauptgefäß freisetzbar sind. Über diese, im Hauptgefäß befindlichen Seitenäste der Gefäßprothese können bspw. weitere Stents/Stentgrafts angebracht werden, welche dann in abzweigende Gefäße eingeführt werden, um die Blutversorgung dorthin zu gewährleisten.

Durch den verringerten Durchmesser im mittleren Gefäßprothesenabschnitt wird dabei sichergestellt, dass sich die in diesem Bereich erstreckenden Seitenäste gut expandieren können und gute Andockstellen für anzuschließende und in Seitengefäße abgehende Stents/Stentgrafts bilden. Gleichzeitig wird über die zweite Öffnung des Gefäßprothesenrumpfes, welche einen im Vergleich zur ersten Öffnung verringerten Durchmesser aufweist, nach wie vor das Hauptgefäß mit Blut versorgt.

Dabei bedeutet "nur in einen Umfangsteil - bzw. Teil des Umfangs -, und nicht über den gesamtem jeweiligen Umfang hin verjüngen" in Bezug auf den ersten und den zweiten mittleren Gefäßprothesenrumpf-Längsabschnitt, dass die mittleren Gefäßprothesenrumpfabschnitte sich nicht über deren jeweiligen gesamten umlaufenden Umfang hin zum zweiten distalen Gefäßprothesenrumpf-Längsabschnitt hin verjüngen, sondern nur über bzw. in einem Abschnitt des Umfangs, also eines Teils des Umfangs. Dadurch weist die erfindungsgemäße Gefäßprothese in den mittleren Gefäßprothesenrumpfabschnitten sozusagen jeweils zwei "Seiten" auf, nämlich eine Seite, in der sich der jeweils mittlere Gefäßprothesenabschnitt verjüngt, und eine Seite, in der sich der jeweils mittlere Gefäßprothesenabschnitt nicht verjüngt.

Der sich verjüngende Teil nimmt dabei vorzugsweise zwischen einem Drittel und zwei Drittel, noch bevorzugter in etwa die Hälfte des Umfangs der jeweiligen ersten und zweiten mittleren Gefäßprothesenrumpf-Längsabschnitte ein.

Dabei ist bevorzugt, wenn der sich verjüngende Umfangsteil des ersten mittleren Gefäßprothesen-Längsabschnitts in den sich nicht verjüngenden Umfangsteil des sich distal anschließenden zweiten mittleren Gefäßprothesen- Längsabschnitts übergeht, und wenn der sich nicht verjüngende Umfangsteil des ersten mittleren Gefäßprothesen-Längsabschnitts in den sich verjüngenden Umfangteil des sich distal anschließenden zweiten mittleren Gefäßprothesen- Längsabschnitts übergeht.

Aufgrund der Bauweise der erfindungsgemäßen Gefäßprothese, drücken die Seitenäste, die sich im Bereich der zweiten, distalen Öffnung nach distale erstrecken, diese nicht zusammen, sobald sei expandiert sind. Ein weiterer Vorteil ist, dass bei Expansion der Seitenäste die Gefäßwand in diesem Bereich nicht mit zusätzlichen Drücken belastet wird, die im Falle eines gleichmäßigen Durchmessers des Gefäßprothesenrumpfes im Bereich der angehenden Seitenäste auftreten würden.

Vorliegend ist mit "Gefäßprothesenrumpf" der Hauptkörper der zylinderförmigen Gefäßprothese gemeint, von welchem Seitenäste abgehen, die sich in distale Richtung und parallel zur axialen Längsachse der Gefäßprothese erstrecken.

Die Prothese ist insgesamt einstückig ausgebildet; hierzu weist die erfindungsgemäße Prothese ein Prothesenmaterial auf, an das jeweils einzelne, mäanderförmig umlaufende Stentringe angebracht, vorzugsweise vernäht, sind; die Stentringe sind dabei nicht direkt miteinander verbunden, sondern nur über das einstückige Prothesenmaterial. Die Stentringe weisen in proximale und distale Richtung weisende Spitzbögen auf. Ferner sind der oder die Seitenäste an den Gefäßprothesenrumpf befestigt.

Grundsätzlich werden bei Gefäßprothesen oder endoluminalen Stentgrafts allgemein sowie vorliegend zur Bezeichnung der jeweiligen Enden die Begriffe "distal" und "proximal" verwendet, wobei der Begriff "distal" der Teil bzw. das Ende bezeichnet wird, der/das in Bezug auf den Blutstrom weiter stromabwärts liegt. Der Begriff "proximal" hingegen bezeichnet, wieder in Bezug auf den Blutstrom, einen Teil bzw. das Ende, der/das in Bezug auf den Blutstrom weiter stromaufwärts liegt. Anders ausgedrückt bedeutet der Begriff "distal" in Richtung des Blutstroms, und der Begriff "proximal" der Richtung des Blutstroms entgegengesetzt. Bei Kathetern hingegen, bzw. Einführsystemen bezeichnet der Begriff "distal" das Ende des Katheters bzw. Einführsystems, das in den Patienten eingeführt wird, bzw. das vom Anwender ausgesehen am Entferntesten liegt, und der Begriff "proximal" das Ende, das sich dem Anwender näher zugewandt ist.

Vorliegend ist daher definitionsgemäß der zweite mittlere Gefäßprothesenrumpf-Längsabschnitt distal bzw. in distale Richtung dem ersten mittleren Gefäßprothesenrumpf-Längsabschnitt unmittelbar nachgeordnet.

Gemäß einer bevorzugten Ausführungsform der erfindungsgemäßen selbstexpandierenden Gefäßprothese ist im ersten mittleren Gefäßprothesenrumpf-Längsabschnitt mindestens ein Gefäßprothesen-Seitenast angeordnet, der sich nach distal parallel zur axialen Längsachse des hohlzylindrischen Grundkörpers erstreckt. Gemäß einer weiteren Ausführungsform der erfindungsgemäßen selbstexpandierenden Gefäßprothese ist im zweiten mittleren Gefäßprothesenrumpf-Längsabschnitt mindestens ein Gefäßprothesen-Seitenast angeordnet, der sich nach distal parallel zur axialen Längsachse des hohlzylindrischen Grundkörpers erstreckt. Mit "mindestens ein Seitenast" ist/sind dabei vorliegend vorzugsweise ein, zwei, drei oder vier Seitenäste gemeint.

Gemäß einer weiteren Ausführungsform der selbstexpandierenden Gefäßprothese sind in den beiden mittleren Gefäßprothesenrumpf-Längsabschnitten insgesamt vier Gefäßprothesen-Seitenäste angeordnet sind. Diese sind dabei vorzugsweise und gemäß einer weiteren Ausführungsform derart am Gefäßprothesenrumpf angebracht, dass sich jeweils zwei in im ersten mittleren Gefäßprothesenrumpf-Längsabschnitt über dessen Umfang verteilt befinden, und zwar im sich verjüngenden Umfangsteil, und die zwei anderen im zweiten mittleren Gefäßprothesenrumpf-Längsabschnitt, ebenfalls über dessen Umfang verteilt, in dem sich verjüngenden Umfangsteil des zweiten mittleren Gefäßprothesenrumpf-Längsabschnitts.

In einer Weiterbildung der selbstexpandierenden Gefäßprothese gemäß der vorliegenden Erfindung ist der erste, proximale Gefäßprothesenrumpf-Längsabschnitt länger als die beiden mittleren Gefäßprothesenrumpf-Längsabschnitte und/oder als der zweite, distale Gefäßprothesenrumpf-Längsabschnitt. In anderen Worten bedeutet dies, dass der erste Gefäßprothesenrumpf-Längsabschnitt dabei eine Länge aufweist, die größer ist als die Länge sowohl des mittleren Gefäßprothesenrumpf-Längsabschnitts als auch des zweiten, distalen Gefäßprothesenrumpf-Längsabschnitts. In einer weiteren Ausführungsform ist die Länge des ersten, proximalen Gefäßprothesenrumpf-Längsabschnitts länger als die Längen des mittleren und zweiten, distalen Gefäßprothesenrumpf-Längsabschnitts zusammen.

In einer bevorzugten Ausführungsform besitzt der erste, proximale Gefäßprothesenrumpf-Längsabschnitt eine Länge von ca. 40 mm bis ca. 100 mm, vorzugswiese ca. 50, 60, 70 oder ca. 80 mm, der erste mittlere Gefäßprothesenrumpf-Längsabschnitt eine Länge von ca. 10 mm bis ca. 30 mm, vorzugsweise ca. 10, 15, 20, 25 mm, der zweite mittlere Gefäßprothesenrumpf-Längsabschnitt eine Länge von ca. 8 mm bis ca. 25 mm, vorzugsweise ca. 8, 10, 12, 14, 16, 18, oder 20 mm, und der zweite, distale Gefäßprothesenrumpf-Längsabschnitt eine Länge von ca. 10 mm bis ca. 50 mm, vorzugsweise ca. 20, 30, oder 40 mm. Dem Fachmann wird dabei klar sein, dass es auf die Gegebenheiten der jeweiligen Gefäße ankommt, die mit der erfindungsgemäßen Prothese behandelt werden sollen. Die Gesamtlänge der erfindungsgemäßen Gefäßprothese kann dabei zwischen 100 und 140 mm, vorzugsweise 110, 120 oder 130 mm.

Sämtliche Größenangaben, die in der vorliegenden Erfindung mit "ca." spezifiziert sind, bedeuten, dass nicht nur der jeweils ganz konkret angegebene Wert umfasst sein soll, sondern auch leicht Abweichungen nach oben oder unten, die im Bereich der Fertigungstoleranzen liegen. Beispielhaft ist mit "ca." gemeint, dass die Größe jeweils ± 0,5 mm vom angegebenen Wert abweichen kann.

Der Durchmesser des ersten proximalen Gefäßprothesenrumpf-Längsabschnitts kann dabei eine Größe von ca. 24 bis 50 mm aufweisen, der Durchmesser des zweiten distalen Gefäßprothesenrumpf-Längsabschnitts eine Größe von ca. 10 bis 30 mm. Der Durchmesser der Seitenäste kann zwischen 5 und 10, vorzugsweise zwischen 6 und 9 mm betragen, wobei der Durchmesser der Seitenäste im zweiten mittleren Gefäßprothesenrumpf-Längsabschnitt in der Regel kleiner ist als der Seitenäste im ersten mittleren Gefäßprothesenrumpf-Längsabschnitt. So sind beispielhafte Werte für den Durchmesser des/der Seitenäste des ersten mittleren Gefäßprothesenrumpf-Längsabschnitts 8 mm, und gleichzeitig der Durchmesser des/der Seitenäste des zweiten mittleren Gefäßprothesenrumpf-Längsabschnitts 6 mm, der Durchmesser für den ersten proximalen Gefäßprothesenrumpf-Längsabschnitt - und damit auch für die erste Öffnung - ca. 36 mm, und der Durchmesser für den zweiten distalen Gefäßprothesenrumpf-Längsabschnitt - und damit auch für die zweite Öffnung - ca. 18 mm.

In einer Weiterbildung der erfindungsgemäßen Gefäßprothese sind im ersten und im zweiten mittleren Gefäßprothesenrumpf-Längsabschnitt jeweils zwei Gefäßprothesen-Seitenäste vorgesehen, wobei die zwei Gefäßprothesen-Seitenäste des bzw. im ersten mittleren Gefäßprothesenrumpf-Längsabschnitt(s) in Bezug auf den Umfang des Gefäßprothesenrumpfs versetzt zu den zwei Gefäßprothesen-Seitenästen des zweiten mittleren Gefäßprothesenrumpf-Längsabschnitts angesetzt bzw. angeordnet sind.

Vorliegend und nachstehend bedeutet dabei "angesetzt", dass die Seitenäste auf dem jeweiligen Umfang des mittleren Gefäßprothesenrumpf-Längsabschnitts über eines ihrer zwei Enden an dem Gefäßprothesenrumpf fixiert sind, während das jeweilige zweite Ende der Seitenäste frei ist.

Gemäß einer weiteren Ausführungsform der selbstexpandierenden Gefäßprothese sind die zwei Gefäßprothesen-Seitenäste des ersten mittleren Gefäßprothesenrumpf-Längsabschnitts in Bezug auf eine radiale Achse, die senkrecht zu der durch den ersten und zweiten konzentrisch angeordneten Gefäßprothesenrumpf-Längsabschnitt verlaufenden axialen Längsachse verläuft, im sich verjüngenden Umfangsteil jeweils rechts und links der Achse in einem Winkel von jeweils zwischen ca. 30° und 60°, vorzugsweise ca. 45°, angeordnet.

Diese Ausführungsform hat den Vorteil, dass durch die versetzte Verteilung der abgehenden Seitenäste über den Umfang des Gefäßprothesenrumpfes sozusagen eine gleichmäßige Materialverteilung im Gefäß erreicht wird, bzw. eine etwaige Materialanhäufung an einer Stelle des Gefäßprothesenrumpfes vermieden wird.

Gemäß einer weiteren Ausführungsform der selbstexpandierenden Gefäßprothese gemäß der vorliegenden Erfindung sind die zwei Gefäßprothesen-Seitenäste des zweiten mittleren Gefäßprothesenrumpf-Längsabschnitts in Bezug auf eine radiale Achse, die senkrecht zu der durch den ersten und zweiten konzentrisch angeordneten Gefäßprothesenrumpf-Abschnitt verlaufenden axialen Längsachse verläuft, im sich verjüngenden Umfangsteil jeweils rechts und links der radialen Achse in einem Winkel von jeweils zwischen ca. 120° und 150°, vorzugsweise ca. 135°, angesetzt bzw. angeordnet.

Auch mit dieser Ausführungsform kann eine Materialanhäufung vermieden werden.

Wie weiter oben erwähnt, weist gemäß einer weiteren Ausführungsform der erfindungsgemäßen selbstexpandierenden Gefäßprothese der Gefäßprothesenrumpf und/oder die Gefäßprothesen-Seitenäste in Längsrichtung in einem Abstand hintereinander angeordnete, jeweils mäanderförmig umlaufende Stentfedern oder Stentringe auf, welche Stentfedern/-ringe nur bzw. ausschließlich über ein Prothesenmaterial, und nicht direkt - bspw. über Stege oder Verbindungspunkte - unter- oder miteinander verbunden sind.

Wie ebenfalls weiter oben erwähnt, weist die erfindungsgemäße Gefäßprothese gemäß einer bevorzugten Ausführungsform Stents auf, die auf der der Gefäßwand zugewandten Seite über ein biokompatibles Graft- oder Prothesenmaterial miteinander verbunden sind. Die Stents stellen mäanderförmig umlaufende Stentringe aus einem selbstexpandierendem Material mit Shape-Memory dar, vorzugsweise Nitinol, die abwechselnd in proximale und distale Richtung weisende Spitzbögen oder Stützen aufweisen. Auch die Seitenäste können auf diese Weise aufgebaut sein. Eine nähere Beschreibung solcher Stents sowie des Graft-/Prothesenmaterials findet sich bspw. in der DE 103 37 739, auf deren gesamten Inhalt hiermit spezifisch Bezug genommen wird.

Allgemein ist bei dem erfindungsgemäßen Gefäßprothese bevorzugt, wenn das Prothesenmaterial ein Material aufweist, das ausgewählt ist aus einem Textil oder einem Polymer.

Insbesondere ist bevorzugt, wenn das Prothesenmaterial ein Material aufweist oder aus diesem gebildet ist, das ausgewählt ist aus Polyester, Polyurethan, Polystyrol, Polytetrafluorethylen ultrahochmolekulargewichtiges Polyethylen (UHMPE), oder Mischungen davon.

Die erfindungsgemäße Gefäßprothese weist dabei in einer Ausführungsform im ersten proximalen und im zweiten distalen Gefäßprothesenabschnitt zumindest einen, vorzugsweise zwei oder drei Stentringe mit gleichmäßig, d.h. mit gleicher Amplitude, in proximale und distale Richtung ausschlagenden Spitzbögen auf, die abwechselnd in distale und proximale Richtung weisen. Gemäß einer weiteren Ausführungsform ist der am proximalen Ende der Gefäßprothese angebrachte Stentring nur über seine nach distal weisenden Spitzbögen, nicht aber seine nach proximal weisenden Spitzbögen am Prothesenmaterial befestigt.

Gemäß einer weiteren Ausführungsform weisen die mittleren Gefäßprothesenrumpf-Längsabschnitte Stentringe mit unterschiedlichen, d.h. mit unterschiedlicher Amplitude in die distale und proximale Richtung ausschlagende Spitzbögen auf. Die sich verjüngenden Umfangsteile können bspw. durch engere Stentringe in diesem Bereich bewirkt werden. Der erste mittlere Gefäßprothesenrumpf-Längsabschnitt weist in einer bevorzugten Ausführungsform zwei solcher Stentringe auf, und der zweite mittlere Gefäßprothesenrumpf-Längsabschnitt einen solchen Stentring. Auch die Seitenäste können in einer bevorzugten Ausführungsform zumindest einen, vorzugsweise einen, Stentring mit unterscheidlicher Amplitude der Spitzbögen aufweisen.

In einer Weiterbildung der erfindungsgemäßen Gefäßprothese sind zumindest im Bereich der jeweiligen Gefäßprothesenrumpf-Enden und/oder der Gefäßprothesen-Seitenästen-Enden und/oder im Bereich des Abgangs der Gefäßprothesen-Seitenäste von dem Gefäßprothesenrumpf röntgendichte Marker vorgesehen sind.

Vorzugsweise sind die röntgendichten Marker aus einem oder mehreren der folgenden Materialien, bspw. Gold, Palladium, Tantal, Chrom, Silber, etc.; die Form der Marker kann dabei beliebig sein, bspw. rund, eckig, und/oder bspw. die Form von Buchstaben, Zahlen oder Figuren haben, die für die Orientierung der Prothese im Gefäß hilfreich sind.

Gemäß einer weiteren Ausführungsform der erfindungsgemäßen Gefäßprothese sind die vom Gefäßprothesenrumpf abgehenden Gefäßprothesen-Seitenäste über ovale Öffnungen im sich jeweils verjüngenden Umfangsteil des Gefäßprothesenrumpfs mit diesem verbunden.

In einer weiteren Ausführungsform ist/sind ferner der Gefäßprothesenrumpf zur Platzierung in der thorakalen-abdominalen Aorta und die Gefäßprothesen-Seitenäste zur Versorgung der Viszeralarterien ausgebildet.

Gemäß einer Weiterbildung der erfindungsgemäßen Gefäßprothese umfasst diese ferner zumindest einen ersten Stentgraft, mit einer ersten und einer zweiten Stentgraft-Öffnung, sowie mit einem sich zwischen den Stentgraft-Öffnungen erstreckenden Lumen, wobei die erste Stentgraft-Öffnung zur teilweisen Einführung in die zweite Öffnung der Gefäßprothese ausgebildet und dimensioniert ist.

In einer weiteren Ausführungsform umfasst die erfindungsgemäße Gefäßprothese ferner zumindest eine zweite Gefäßprothese, mit einer ersten und einer zweiten Gefäßprothesen-Öffnung, sowie mit einem sich zwischen den Gefäßprothesen-Öffnungen erstreckenden Lumen, wobei die erste Gefäßprothesen-Öffnung zur teilweisen Einführung in die Gefäßprothesen-Seitenäste ausgebildet ist, zur Versorgung der von einem Hauptgefäß abgehenden Seitengefäße.

Bei dieser Ausführungsform ist bevorzugt, wenn die zweite Gefäßprothese gestentet oder ungestentet ist.

Weitere Vorteile ergeben sich aus den Figuren und der nachstehenden Beschreibung bevorzugter Ausführungsbeispiele.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:

- Fig. 1: eine schematische Darstellung einer Ausführungsform einer erfindungsgemäßen Gefäßprothese, im nicht-eingeführten, expandierten Zustand in perspektivischer Ansicht der Längsseite von seitlich-oben;
- Fig. 2: die Ausführungsform aus Fig. 1 von der andren Seite;
- Fig. 3: eine andere schematische Darstellung der Ausführungsform aus Fig. 1, in voller seitlicher Ansicht der Längsseite, mit den Stentringen schematisch eingezeichnet;
- Fig. 4: die gleiche schematische Darstellung wie in Fig. 3, in Längsansicht von oben; und
- Fig. 5: eine schematische Darstellung der in den Fig. 1 bis 4 gezeigten Ausführungsform in frontaler Ansicht durch das distale Ende zum proximalen Ende.

In den Figuren werden gleiche Merkmale mit gleichen Bezugszeichen versehen, wobei aus Gründen der Übersichtlichkeit nicht in allen Figuren immer sämtliche Bezugszeichen angegeben sind.

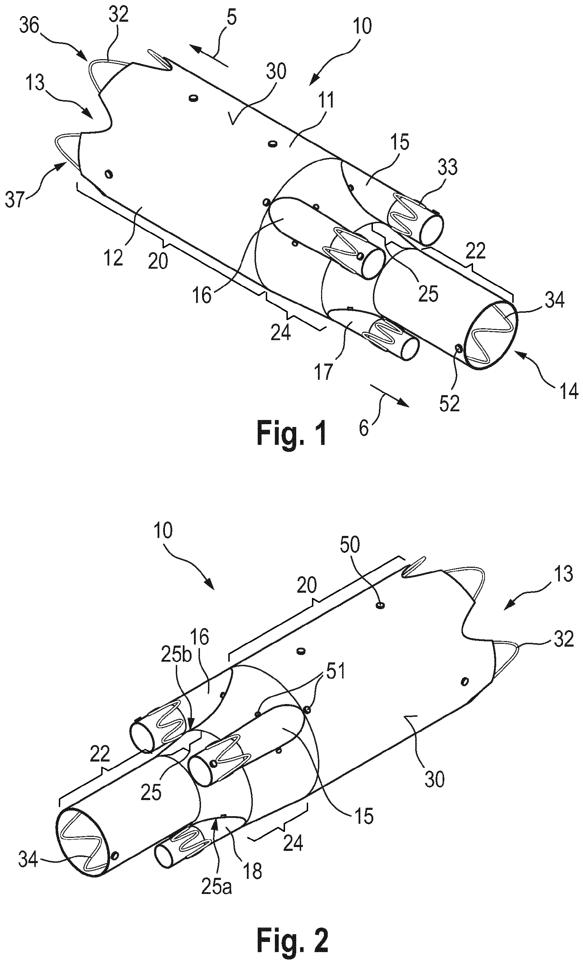

In den Figuren 1 bis 4 ist mit 10 insgesamt eine Gefäßprothese bezeichnet, mit einem generell hohlzylindrischen Grundkörper 11, sowie einem Gefäßprothesenrumpf 12 mit einer ersten, proximalen Öffnung 13 und einer zweiten, distalen Öffnung 14. Die Gefäßprothese 10 weist ferner vom Gefäßprothesenrumpf 12 abgehende Seitenäste 15, 16, 17 und 18 auf, wobei in Fig. 1 aufgrund der der perspektivischen Darstellung der Seitenast 18 nicht gezeigt ist, und in Fig. 2 der Seitenast 17 nicht dargestellt ist.

Die in den Figuren 1 und 2 gezeigte Gefäßprothese weist ferner einen ersten proximalen Gefäßprothesenrumpf-Längsabschnitt 20 aufweist, der die erste Öffnung 13 umfasst, sowie einen zweiten distalen Gefäßprothesenrumpf-Längsabschnitt 22, der die zweite Öffnung 14 umfasst. Den Figuren 1 und 2 ist ferner zu entnehmen, dass die Gefäßprothese 10 außerdem zwei mittlere Gefäßprothesenrumpf-Längsabschnitte 24 und 25 aufweist, die zwischen dem ersten und zweiten Gefäßprothesenrumpf-Längsabschnitt 20, 22 hintereinander angeordnet sind.

Vom ersten mittleren Gefäßprothesenrumpf-Längsabschnitt 24 erstrecken sich die beiden Seitenäste 15 und 16, vom zweiten mittleren Gefäßprothesenrumpf-Längsabschnitt 25 erstrecken sich die beiden Seitenäste 17 und 18, jeweils in distale Richtung, die mit dem Pfeil 6 angedeutet ist.

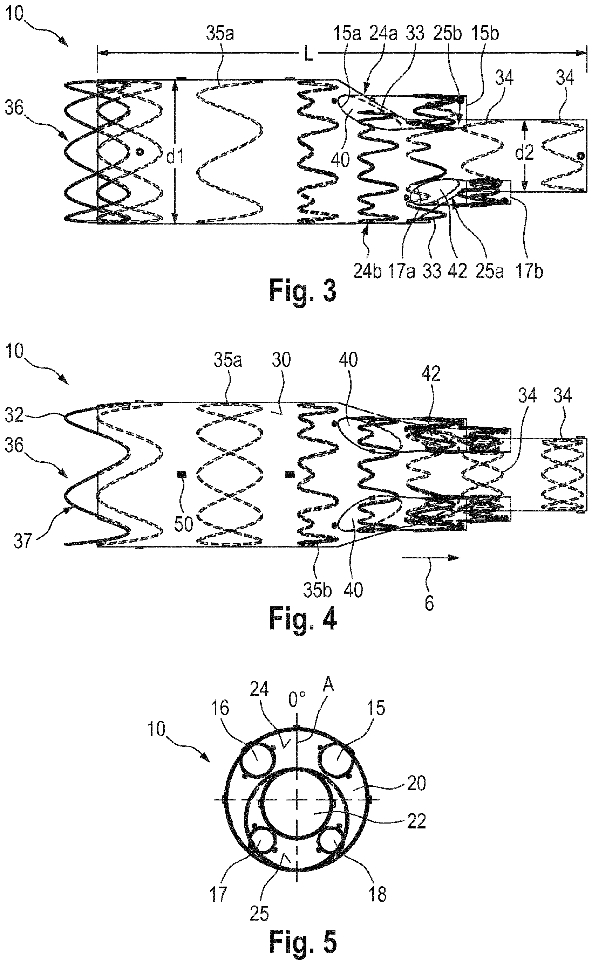

Der erste proximale Gefäßprothesenrumpf-Längsabschnitt 20 und der zweite distale Gefäßprothesenrumpf-Längsabschnitt 22 weisen jeweils über ihre Länge hinweg im Wesentlichen konstante Durchmesser d1 und d2 auf und sind in Bezug aufeinander konzentrisch angeordnet (siehe hierzu auch Fig. 5). Dabei ist der Durchmesser d1 des ersten proximalen Gefäßprothesenrumpf-Längsabschnitts 20 größer als der Durchmesser d2 des zweiten distalen Gefäßprothesenrumpf-Längsabschnitts 22.

Die in den Figuren 1 bis 4 gezeigte Gefäßprothese 10 weist ferner ein sich über die gesamte Länge L der Gefäßprothese 10 erstreckendes Prothesenmaterial 30 auf, an welches Stentringe 32, 33, 34 und 35 angebracht sind. Die Stentringe 32, 33, 34 und 35 stellen mäanderförmig umlaufende, aus einem Draht gebildete und alternierend in die proximale Richtung 5 und distale Richtung 6 weisende Spitzbögen 36 dar, die durch Stützen 37 miteinander verbunden sind. Aus Gründen der Übersichtlichkeit sind in den Fig. 1 und 2 nur die jeweils die Öffnungen 13 und 14 umgebenden Stentringe 32 und 34, sowie die Stentringe 33 der Seitenäste gezeigt.

Der Stentring 32 befindet sich dabei am proximalen Ende der Gefäßprothese 10 und ist umlaufend um die Öffnung 13 von der inneren Seite des hohlzylindrischen Grundkörpers 11 an das Prothesenmaterial 30 angebracht, vorzugsweise angenäht. Der Stentring 32 weist - und dies ist insbesondere auch in den weiter unten beschriebenen Figuren 3 und 4 ersichtlich - Spitzbögen 36 mit gleicher Amplitude auf, d.h. die die Spitzbögen 36 verbindenden Stützen 37 haben sämtlich die gleiche Länge. Fig. 1 und 2 ist zu entnehmen, dass der Stentring 32 lediglich über die in distale Richtung 6 weisenden Spitzbögen 36 an das Prothesenmaterial 30 angebracht ist, sowie über einen Abschnitt der diese verbindenden Stützen 37, so dass die in proximale Richtung 5 weisenden Spitzbögen 36 frei von Prothesenmaterial sind.

Die Stentringe 33 der Seitenäste 15, 16, 17, und 18 sind in der in den Figuren 1 bis 4 gezeigten Ausführungsform von außen auf das Prothesenmaterial 30 fixiert, vorzugsweise angenäht. Diese Stentringe 33 weisen unterschiedliche Amplituden auf, d.h. die die Spitzbögen 36 verbindenden Stützen 37 sind hier unterschiedlich lang.

Der Stentring 34 ist am distalen Ende im zweiten distalen Gefäßprothesenrumpf-Längsabschnitt 22 vorgesehen und ist umlaufend um die Öffnung 14 von der inneren Seite des hohlzylindrischen Grundkörpers 11 an das Prothesenmaterial 30 angebracht, vorzugsweise angenäht. Der Stentring 34 weist - und dies ist insbesondere auch in den weiter unten beschriebenen Figuren 3 und 4 ersichtlich - Spitzbögen 36 mit gleicher Amplitude auf, d.h. die die Spitzbögen 36 verbindenden Stützen 37 haben sämtlich die gleiche Länge. Der Stentring 34 ist dabei über seine gesamte durch ihn definierte Fläche am Prothesenmaterial 30 fixiert.

Sowohl der erste proximale Gefäßprothesenrumpf-Längsabschnitt 20 also auch der zweite distale Gefäßprothesenrumpf-Längsabschnitt 22 können jeweils weitere, Stentringe 32, 33, 34 oder 35 aufweisen, die über die jeweilige Länge des betreffenden Gefäßprothesenrumpf-Längsabschnitts 20, 22 hintereinander in einem bestimmten Abstand angeordnet sind, was insbesondere aus den Figuren 3 und 4 hervorgeht: Hier ist mit dem Bezugszeichen 35a ein weiterer Stentring bezeichnet, der dem Stentring 32 im ersten proximalen Gefäßprothesenrumpf-Längsabschnitt 20 nachgeordnet ist, und ebenfalls von Innen an das Prothesenmaterial 30 fixiert ist. Auch dieser Stentring 35a weist eine gleichmäßige Amplitude auf. Auch der zweite distale Gefäßprothesenrumpf-Längsabschnitt 22 weist einen zweiten Stentring 34 auf, der eine gleichmäßige Amplitude besitzt. Der erste proximale Gefäßprothesenrumpf-Längsabschnitt 20 weist ferner einen Stentring 35b auf, der eine unregelmäßige Amplitude aufweist, d.h. hier weisen die die Spitzbögen 36 verbindenden Stützen unterschiedliche Längen auf.

Den Figuren 3 und 4 ist ferner zu entnehmen, dass der erste mittlere Gefäßprothesenrumpf-Längsabschnitt 24 und der zweite mittlere Gefäßprothesenrumpf-Längsabschnitt 25 jeweils einen Stentring 33 aufweisen, die jeweils ungleichmäßige Amplituden aufweisen.

Den Figuren 1 bis 4 ist ferner zu entnehmen, dass sich der erste und der zweite mittlere Gefäßprothesenrumpf-Längsabschnitt 24, 25 jeweils in distale Richtung in einem ihrer Umfangsteile 24a, 25b sozusagen "einseitig" verjüngen. Dies ist insbesondere der Seitenansicht der Figur 3 zu entnehmen: Hier weist der erste mittlere Gefäßprothesenrumpf-Längsabschnitt 24 in seinem Umfang einen Umfangsteil 24a auf, der - in seitlicher Ansicht - schräg verjüngend in distaler Richtung 6 verläuft, wohingegen der Umfangsteil 24b sich nicht verjüngt und in seitlicher Ansicht daher gerade verläuft. In der Draufsicht, die in Fig. 4 dargestellt ist, ist zu erkennen, dass der sich verjüngende Umfangteil 24a des ersten mittleren Gefäßprothesenrumpf-Längsabschnitts 24 ca. die Hälfte des Umfangs des mittleren Gefäßprothesenrumpf-Längsabschnitts 24 einnimmt. In diesem sich verjüngenden Umfangsteil 24a befinden sich ovale Öffnungen 40 im Prothesenmaterial 30 der Gefäßprothese 10. Über diese ovalen Öffnungen 40 sind die Seitenäste 15 und 16 mit ihrem mit dem Gefäßprothesenrumpf verbundenen Ende 15a angebracht, während das Ende 15b sich frei in distale Richtung 6 erstreckt.

Figur 1 und 2 ist zu entnehmen, dass auch der zweite mittlere Gefäßprothesenrumpf-Längsabschnitt 25 in seinem Umfang einen Umfangsteil 25a aufweist, derin seitlicher Ansicht - schräg verjüngend in distaler Richtung 6 verläuft, wohingegen der Umfangsteil 25b sich nicht verjüngt und in seitlicher Ansicht daher gerade verläuft. In den seitlichen Ansichten der Figuren 1 und 2 ist zu erkennen, dass der sich verjüngende Umfangteil 25a des zweiten mittleren Gefäßprothesenrumpf-Längsabschnitts 25 ca. die Hälfte des Umfangs des zweiten mittleren Gefäßprothesenrumpf-Längsabschnitts 25 einnimmt. Auch in diesem sich verjüngenden Umfangsteil 25a befinden sich ovale Öffnungen 42 (siehe Figur 3 und 4) im Prothesenmaterial 30 der Gefäßprothese 10. Über diese ovalen Öffnungen 42 sind die Seitenäste 17 und 18 mit ihrem mit dem Gefäßprothesenrumpf 12 verbundenen Ende 17a angebracht, während das Ende 17b sich frei in distale Richtung 6 erstreckt.

Insbesondere den Figuren 3 und 4 ist ferner zu entnehmen, dass der sich verjüngende Umfangsteil 24a des Gefäßprothesenrumpf-Längsabschnitts 24 in distale Richtung 6 an den sich nicht-verjüngenden Umfangteil 25b des zweiten Gefäßprothesenrumpf-Längsabschnitts 25 unmittelbar anschließt. Auf der anderen Seite geht der sich nicht-verjüngende Umfangsteil 24b des ersten mittleren Gefäßprothesenrumpf-Längsabschnitts 24 in den sich verjüngenden Umfangsteil 25a des zweiten mittleren Gefäßprothesenrumpf-Längsabschnitts 25 über.

In den Figuren 1 bis 4 sind ferner röntgendichte Marker 50, 51, 52 vorgesehen, wobei die Marker 50 in dem in den Figuren 1 bis 4 gezeigten Ausführungsbeispiel mittig und parallel der Längsachse der Gefäßprothese 10 auf dem Prothesenmaterial 30 in einem bestimmten Abstand hintereinander angeordnet sind. Die Marker 51 sind um die ovalen Öffnungen 40, 42 herum angeordnet, um die abgehenden Seitenäste15, 16, 17 und 18 zu markieren. Der Marker 52 ist im Bereich bzw. an der zweiten, distalen Öffnung 14 angebracht, um dem Operateur diese in der Röntgenkontrolle entsprechend anzuzeigen.

Figur 5 schließlich zeigt die Draufsicht in das Lumen des hohlzylindrischen Grundköpers 11, und zwar mit Einblick in die zweite, distale Öffnung 14 in Richtung der ersten proximalen Öffnung 13. Zu erkennen ist hier, dass der erste und der zweite Gefäßprothesenrumpf-Längsabschnitt 20, 22 zum einen unterschiedliche Durchmesser d1 und d2 aufweisen, und darüber hinaus konzentrisch zueinander angeordnet sind.

Dieser Figur ist ferner zu entnehmen, dass die Seitenäste 15 und 16 in Bezug auf eine Achse A, die senkrecht zu der durch den ersten und zweiten konzentrisch angeordneten Gefäßprothesenrumpf-Längsabschnitt 20, 22 verlaufenden Längsachse L im sich verjüngenden Umfangsteil 24b jeweils rechts und links der Achse A in einem Winkel mit ca. 45° angeordnet sind.

Andererseits sind die Seitenäste 17 und 18 in Bezug auf die Achse A im sich verjüngenden Umfangsteil 25b jeweils rechts und links der Achse A in einem Winkel von ca. 135° angeordnet.

Mit der vorteilhaften Ausgestaltung der mittleren Gefäßprothesenrumpf-Längsabschnitte 24, 25 sowie der sich hieraus ergebenden Möglichkeit, die Seitenäste Material- und Platzsparend anzubringen.

## Patentansprüche

1. Selbstexpandierende Gefäßprothese (10) zur Implantation in ein Blutgefäß eines Patienten, mit einem einen hohlzylindrischen Grundkörper (11), sowie einem eine erste und eine zweite Öffnung (13, 14) aufweisenden Gefäßprothesenrumpf (12) und mit mindestens zwei vom Gefäßprothesenrumpf (12) abgehenden Gefäßprothesen-Seitenästen (15, 16, 17, 18), die einstückig mit dem Gefäßprothesenrumpf (12) ausgebildet sind, wobei der Gefäßprothesenrumpf (12)
- einen ersten proximalen Gefäßprothesenrumpf-Längsabschnitt (20), der die erste Öffnung (13) umfasst, einen zweiten distalen Gefäßprothesenrumpf-Längsabschnitt (22), der die zweite Öffnung (14) umfasst, und zumindest zwei zwischen dem ersten und dem zweiten Gefäßprothesenrumpf-Längsabschnitt (20, 22) angeordneten mittlere Gefäßprothesenrumpf-Längsabschnitte (24, 25) aufweist, die hintereinander angeordnet sind, und wobei die mindestens zwei vom Gefäßprothesenrumpf (12) abgehenden Gefäßprothesen-Seitenäste (15, 16, 17, 18) in den mittleren Gefäßprothesenrumpf-Längsabschnitten (24, 25) angeordnet sind,
- wobei der erste und der zweite Gefäßprothesenrumpf-Längsabschnitt (20, 22) jeweils einen konstanten Durchmesser d1, d2 über ihre jeweilige Längsrichtung aufweisen und in Bezug aufeinander konzentrisch angeordnet sind, und wobei der Durchmesser d1 des ersten Gefäßprothesenrumpf-Längsabschnitts (20) größer ist als der Durchmesser d2 des zweiten Gefäßprothesenrumpf-Längsabschnitts (22),
**dadurch gekennzeichnet, dass**
sich der erste und der zweite mittlere Gefäßprothesenrumpf-Längsabschnitt (24, 25) jeweils zum zweiten Gefäßprothesenrumpf-Längsabschnitt (22) nur in einen Umfangsteil (24a, 25a) hin verjüngen, wobei die sich jeweils verjüngenden Umfangsteile (24a, 25a) des ersten und des zweiten mittleren Gefäßprothesenrumpf-Längsabschnitts (24, 25) in Umfangsrichtung versetzt zueinander angeordnet sind, und dass zumindest ein Gefäßprothesen-Seitenast (15, 16) im ersten (24) und zumindest ein zweiter Gefäßprothesen-Seitenast (17, 18) im zweiten (25) mittleren Gefäßprothesenrumpf-Längsabschnitt in dem sich jeweils verjüngenden Umfangsteil (24a, 25a) angeordnet sind.

2. Selbstexpandierende Gefäßprothese (10) nach Anspruch 1, **dadurch gekennzeichnet, dass** im ersten mittleren Gefäßprothesenrumpf-Längsabschnitt (24) mindestens ein Gefäßprothesen-Seitenast (15, 17) angeordnet ist, der sich nach distal parallel zur Längsachse des hohlzylindrischen Grundkörpers (11) erstreckt.

3. Selbstexpandierende Gefäßprothese (10) nach Anspruch 1 oder 2, **dadurch gekennzeichnet, dass** im zweiten mittleren Gefäßprothesenrumpf-Längsabschnitt (25) mindestens ein Gefäßprothesen-Seitenast (17, 18) angeordnet ist, der sich nach distal parallel zur Längsachse des hohlzylindrischen Grundkörpers (11) erstreckt.

4. Selbstexpandierende Gefäßprothese (10) nach einem der vorstehenden Ansprüche, **dadurch gekennzeichnet, dass** in den beiden mittleren Gefäßprothesenrumpf-Längsabschnitten (24, 25) insgesamt vier Gefäßprothesen-Seitenäste (15, 16, 17, 18) angeordnet sind.

5. Selbstexpandierende Gefäßprothese (10) nach einem der vorstehenden Ansprüche, **dadurch gekennzeichnet, dass** der erste Gefäßprothesenrumpf-Längsabschnitt (20) länger ist als die beiden mittleren Gefäßprothesenrumpf-Längsabschnitte (24, 25) und/oder als der zweite Gefäßprothesenrumpf-Längsabschnitt (22).

6. Selbstexpandierende Gefäßprothese (10) nach einem der vorstehenden Ansprüche, **dadurch gekennzeichnet, dass** im ersten und im zweiten mittleren Gefäßprothesenrumpf-Längsabschnitt (24, 25) jeweils zwei Gefäßprothesen-Seitenäste (15, 16; 17, 18) vorgesehen sind, wobei die zwei Gefäßprothesen-Seitenäste (15, 16) des ersten mittleren Gefäßprothesenrumpf-Längsabschnitts (24) in Bezug auf den Umfang des Gefäßprothesenrumpfs (12) versetzt zu den zwei Gefäßprothesen-Seitenästen (17, 18) des zweiten mittleren Gefäßprothesenrumpf-Längsabschnitts (25) angeordnet sind.

7. Selbstexpandierende Gefäßprothese (10) nach einem der Ansprüche 2 bis 6, **dadurch gekennzeichnet, dass** die zwei Gefäßprothesen-Seitenäste (15, 16) des ersten mittleren Gefäßprothesenrumpf-Längsabschnitts (24) in Bezug auf eine Achse (A), die senkrecht zu der durch den ersten und zweiten konzentrisch angeordneten Gefäßprothesenrumpf-Abschnitt (20, 22) verlaufenden Längsachse (L) verläuft, im sich verjüngenden Umfangsteil (24a) jeweils rechts und links der Achse (A) in einem Winkel von jeweils zwischen ca. 30° und 60°, vorzugsweise ca. 45°, angeordnet sind.

8. Selbstexpandierende Gefäßprothese (10) nach einem der Ansprüche 3 bis 7, **dadurch gekennzeichnet, dass** die zwei Gefäßprothesen-Seitenäste (17, 18) des zweiten mittleren Gefäßprothesenrumpf-Längsabschnitts (25) in Bezug auf eine Achse(A), die senkrecht zu der durch den ersten und zweiten konzentrisch angeordneten Gefäßprothesenrumpf-Abschnitt (20, 22) verlaufenden Längsachse (L) verläuft, im sich verjüngenden Umfangsabschnitt (25a) jeweils rechts und links der Achse (A) in einem Winkel von jeweils zwischen ca. 120° und 150°, vorzugsweise ca. 135°, angeordnet sind.

9. Selbstexpandierende Gefäßprothese (10) nach einem der vorstehenden Ansprüche, **dadurch gekennzeichnet, dass** der Gefäßprothesenrumpf (12) und/oder die Gefäßprothesen-Seitenäste (15, 16, 17, 18) in Längsrichtung in einem Abstand hintereinander angeordnete, jeweils mäanderförmig umlaufende Stentringe (32, 33, 34, 35) aufweisen, welche Stentringe (32, 33, 34, 35) nur über ein Prothesenmaterial (30) miteinander verbunden sind.

10. Selbstexpandierende Gefäßprothese (10) nach einem der vorstehenden Ansprüche, **dadurch gekennzeichnet, dass** zumindest im Bereich der Öffnungen (13, 14) und/oder der freien Gefäßprothesen-Seitenästen-Enden (15b,17b) und/oder im Bereich des Abgangs (15a, 15b) der Gefäßprothesen-Seitenäste von dem Gefäßprothesenrumpf röntgendichte Marker (50, 51,52) vorgesehen sind.

11. Selbstexpandierende Gefäßprothese (10) nach einem der vorstehenden Ansprüche, **dadurch gekennzeichnet, dass** die vom Gefäßprothesenrumpf (12) abgehenden Gefäßprothesen-Seitenäste (15, 16, 17, 18) über ovale Öffnungen (40, 42) im sich verjüngenden Umfangsteil (24a, 25a) des ersten und/oder zweiten Gefäßprothesenrumpf-Längsabschnitts (24, 25) mit dem Gefäßprothesenrumpf (12) verbunden sind.

12. Selbstexpandierende Gefäßprothese (10) nach einem der vorstehenden Ansprüche, **dadurch gekennzeichnet, dass** der Gefäßprothesenrumpf (12) zur Platzierung in der thorakalen-abdominalen Aorta und die Gefäßprothesen-Seitenäste (15, 16, 17, 18) zur Versorgung der Viszeralarterien ausgebildet ist/sind.

13. Selbstexpandierende Gefäßprothese (10) nach einem der vorstehenden Ansprüche, **dadurch gekennzeichnet, dass** sie ferner zumindest einen ersten Stentgraft umfasst, mit einer ersten und einer zweiten Stentgraft-Öffnung, sowie mit einem sich zwischen den Stentgraft-Öffnungen erstreckenden Lumen, wobei die erste Stentgraft-Öffnung zur teilweisen Einführung in die zweite Öffnung (14) der Gefäßprothese (10) ausgebildet und dimensioniert ist.

14. Selbstexpandierende Gefäßprothese (10) nach einem der vorstehenden Ansprüche, **dadurch gekennzeichnet, dass** sie ferner zumindest eine zweite Gefäßprothese umfasst, mit einer ersten und einer zweiten Gefäßprothesen-Öffnung, sowie mit einem sich zwischen den Gefäßprothesen-Öffnungen erstreckenden Lumen, wobei die erste Gefäßprothesen-Öffnung zur teilweisen Einführung in die Gefäßprothesen-Seitenäste (15, 16, 17, 18) ausgebildet ist, zur Versorgung der von einem Hauptgefäß abgehenden Seitengefäße.

15. Selbstexpandierende Gefäßprothese (10) nach Anspruch 14, **dadurch gekennzeichnet, dass** die zweite Gefäßprothese gestentet oder ungestentet ist.

## Claims

1. A self-expanding vascular prosthesis (10) for implantation in a blood vessel of a patient, with a vascular prosthesis trunk (12) having a hollow-cylindrical main body (11) and also a first and a second opening (13, 14), and with at least two vascular prosthesis side branches (15, 16, 17, 18) which branch off from the vascular prosthesis trunk (12) and are formed integrally with the vascular prosthesis trunk (12), wherein the vascular prosthesis trunk (12) has
- a first, proximal vascular prosthesis trunk longitudinal portion (20), which comprises the first opening (13), a second, distal vascular prosthesis trunk longitudinal portion (22), which comprises the second opening (14), and at least two central vascular prosthesis trunk longitudinal portions (24, 25) which are arranged between the first and second vascular prosthesis trunk longitudinal portions (20, 22) and which are arranged one behind the other, and wherein the at least two vascular prosthesis side branches (15, 16, 17, 18) branching off from the vascular prosthesis trunk (12) are arranged in the central vascular prosthesis trunk longitudinal portions (24, 25),
- wherein the first and second vascular prosthesis trunk longitudinal portions (20, 22) each have a constant diameter d1, d2 along their respective longitudinal direction and are arranged concentrically with respect to each other, and wherein the diameter d1 of the first vascular prosthesis trunk longitudinal portion (20) is greater than the diameter d2 of the second vascular prosthesis trunk longitudinal portion (22),
**characterized in that**
the first and second central vascular prosthesis trunk longitudinal portions (24, 25) each taper toward the second vascular prosthesis trunk longitudinal portion (22) only in one circumferential part (24a, 25a), wherein the respectively tapering circumferential parts (24a, 25a) of the first and second central vascular prosthesis trunk longitudinal portions (24, 25) are arranged in a manner offset in relation to each other in the circumferential direction,
and **in that** at least one vascular prosthesis side branch (15, 16) in the first central vascular prosthesis trunk longitudinal portion (24) and at least one second vascular prosthesis side branch (17, 18) in the second central vascular prosthesis trunk longitudinal portion (25) are arranged in the respectively tapering circumferential part (24a, 25a).

2. The self-expanding vascular prosthesis (10) as claimed in claim 1, **characterized in that** at least one vascular prosthesis side branch (15, 17) is arranged in the first central vascular prosthesis trunk longitudinal portion (24) and extends in the distal direction and parallel to the longitudinal axis of the hollow-cylindrical main body (11).

3. The self-expanding vascular prosthesis (10) as claimed in claim 1 or 2, **characterized in that** at least one vascular prosthesis side branch (17, 18) is arranged in the second central vascular prosthesis trunk longitudinal portion (25) and extends in the distal direction and parallel to the longitudinal axis of the hollow-cylindrical main body (11).

4. The self-expanding vascular prosthesis (10) as claimed in one of the preceding claims, **characterized in that** a total of four vascular prosthesis side branches (15, 16, 17, 18) are arranged in the two central vascular prosthesis trunk longitudinal portions (24, 25).

5. The self-expanding vascular prosthesis (10) as claimed in one of the preceding claims, **characterized in that** the first vascular prosthesis trunk longitudinal portion (20) is longer than the two central vascular prosthesis trunk longitudinal portions (24, 25) and/or than the second vascular prosthesis trunk longitudinal portion (22).

6. The self-expanding vascular prosthesis (10) as claimed in one of the preceding claims, **characterized in that** two vascular prosthesis side branches (15, 16; 17, 18) are provided respectively in the first and in the second vascular prosthesis trunk longitudinal portions (24, 25), wherein, with respect to the circumference of the vascular prosthesis trunk (12), the two vascular prosthesis side branches (15, 16) of the first central vascular prosthesis trunk longitudinal portion (24) are arranged in a manner offset in relation to the two vascular prosthesis side branches (17, 18) of the second central vascular prosthesis trunk longitudinal portion (25).

7. The self-expanding vascular prosthesis (10) as claimed in one of claims 2 to 6, **characterized in that**, with respect to an axis (A) extending perpendicular to the longitudinal axis (L) extending through the first and second concentrically arranged vascular prosthesis trunk portions (20, 22), the two vascular prosthesis side branches (15, 16) of the first central vascular prosthesis trunk longitudinal portion (24) are arranged in the tapering circumferential part (24a), respectively to the right and left of the axis (A), at an angle of in each case between ca. 30° and 60°, preferably ca. 45°.

8. The self-expanding vascular prosthesis (10) as claimed in one of claims 3 to 7, **characterized in that**, with respect to an axis (A) extending perpendicular to the longitudinal axis (L) extending through the first and second concentrically arranged vascular prosthesis trunk portions (20, 22), the two vascular prosthesis side branches (17, 18) of the second central vascular prosthesis trunk longitudinal portion (25) are arranged in the tapering circumferential part (25a), respectively to the right and left of the axis (A), at an angle of in each case between ca. 120° and 150°, preferably ca.135°.

9. The self-expanding vascular prosthesis (10) as claimed in one of the preceding claims, **characterized in that** the vascular prosthesis trunk (12) and/or the vascular prosthesis side branches (15, 16, 17, 18) have stent rings (32, 33, 34, 35) which are arranged at a distance behind one another in the longitudinal direction and each extend in a meandering formation, which stent rings (32, 33, 34, 35) are connected to one another only via a prosthesis material (30).

10. The self-expanding vascular prosthesis (10) as claimed in one of the preceding claims, **characterized in that** radiopaque markers (50, 51, 52) are provided at least in the region of the openings (13, 14) and/or of the free ends (15b, 17b) of the vascular prosthesis side branches, and/or in the region of emergence (15a, 15b) of the vascular prosthesis side branches from the vascular prosthesis trunk.

11. The self-expanding vascular prosthesis (10) as claimed in one of the preceding claims, **characterized in that** the vascular prosthesis side branches (15, 16, 17, 18) off-branching from the vascular prosthesis trunk (12) are connected to the vascular prosthesis trunk (12) via oval openings (40, 42) in the tapering circumferential part (24a, 25a) of the first and/or second vascular prosthesis trunk longitudinal portion (24, 25).

12. The self-expanding vascular prosthesis (10) as claimed in one of the preceding claims, **characterized in that** the vascular prosthesis trunk (12) is designed for placement in the thoraco-abdominal aorta, and the vascular prosthesis side branches (15, 16, 17, 18) are designed to supply the visceral arteries.

13. The self-expanding vascular prosthesis (10) as claimed in one of the preceding claims, **characterized in that** it moreover comprises at least a first stent graft, with a first and a second stent graft opening, and also with a lumen extending between the stent graft openings, wherein the first stent graft opening is designed and dimensioned for partial insertion into the second opening (14) of the vascular prosthesis (10).

14. The self-expanding vascular prosthesis (10) as claimed in one of the preceding claims, **characterized in that** it moreover comprises at least a second vascular prosthesis, with a first and a second vascular prosthesis opening, and also with a lumen extending between the vascular prosthesis openings, wherein the first vascular prosthesis opening is designed for partial insertion into the vascular prosthesis side branches (15, 16, 17, 18), in order to supply the side vessels off-branching from a main vessel.

15. The self-expanding vascular prosthesis (10) as claimed in claim 14, **characterized in that** the second vascular prosthesis is stented or unstented.

## Revendications

1. Prothèse vasculaire auto-expansible (10) pour l'implantation dans un vaisseau sanguin d'un patient, comprenant une coque de prothèse vasculaire (12) présentant un corps de base cylindrique creux (11), ainsi qu'un une première et une deuxième ouverture (13, 14), et comprenant au moins deux branches latérales (15, 16, 17, 18) de prothèse vasculaire partant de la coque de prothèse vasculaire (12), qui sont réalisées d'une seule pièce avec la coque de prothèse vasculaire (12), la coque de prothèse vasculaire (12)
- présentant une première partie longitudinale proximale (20) de coque de prothèse vasculaire, qui comprend la première ouverture (13), une deuxième partie longitudinale distale (22) de coque de prothèse vasculaire, qui comprend la deuxième ouverture (14), et au moins deux parties longitudinales centrales (24, 25) de coque de prothèse vasculaire disposées entre la première et la deuxième partie longitudinale (20, 22) de coque de prothèse vasculaire, qui sont disposées l'une derrière l'autre, et les au moins deux branches latérales (15, 16, 17, 18) de prothèse vasculaire partant de la coque de prothèse vasculaire (12) étant disposées dans les deux parties longitudinales centrales (24, 25) de coque de prothèse vasculaire,
- où la première et la deuxième partie longitudinale (20, 22) de coque de prothèse vasculaire présentent chacune un diamètre constant d1, d2 sur leur direction longitudinale respective et sont disposées concentriquement l'une par rapport à l'autre, et le diamètre d1 de la première partie longitudinale (20) de coque de prothèse vasculaire étant supérieur au diamètre d2 de la deuxième partie longitudinale (22) de coque de prothèse vasculaire,
**caractérisée en ce que**
la première et la deuxième partie longitudinale centrale (24, 25) de coque de prothèse vasculaire se rétrécissent à chaque fois vers la deuxième partie longitudinale (22) de coque de prothèse vasculaire seulement dans une partie périphérique (24a, 25a), les parties périphériques respectives se rétrécissant (24a, 25a) de la première et de la deuxième partie longitudinale centrale (24, 25) de coque de prothèse vasculaire étant disposées de manière décalée l'une par rapport à l'autre dans la direction périphérique,
et **en ce qu'**au moins une branche latérale (15, 16) de prothèse vasculaire dans la première partie longitudinale centrale (24) de coque de prothèse vasculaire et au moins une deuxième branche latérale (17, 18) de prothèse vasculaire dans la deuxième partie longitudinale centrale (25) de coque de prothèse vasculaire sont disposées dans la partie périphérique respective se rétrécissant (24a, 25a).

2. Prothèse vasculaire auto-expansible (10) selon la revendication 1, **caractérisée en ce qu'**au moins une branche latérale (15, 17) de prothèse vasculaire est disposée dans la première partie longitudinale centrale (24) de coque de prothèse vasculaire, laquelle s'étend dans la direction distale parallèlement à l'axe longitudinal du corps de base cylindrique creux (11).

3. Prothèse vasculaire auto-expansible (10) selon la revendication 1 ou 2, **caractérisée en ce qu'**au moins une branche latérale (17, 18) de prothèse vasculaire est disposée dans la deuxième partie longitudinale centrale (25) de coque de prothèse vasculaire, laquelle s'étend dans la direction distale parallèlement à l'axe longitudinal du corps de base cylindrique creux (11).

4. Prothèse vasculaire auto-expansible (10) selon l'une quelconque des revendications précédentes, **caractérisée en ce qu'**au total quatre branches latérales (15, 16, 17, 18) de prothèse vasculaire sont disposées dans les deux parties longitudinales centrales (24, 25) de coque de prothèse vasculaire.

5. Prothèse vasculaire auto-expansible (10) selon l'une quelconque des revendications précédentes, **caractérisée en ce que** la première partie longitudinale (20) de coque de prothèse vasculaire est plus longue que les deux parties longitudinales centrales (24, 25) de coque de prothèse vasculaire et/ou que la deuxième partie longitudinale (22) de coque de prothèse vasculaire.

6. Prothèse vasculaire auto-expansible (10) selon l'une quelconque des revendications précédentes, **caractérisée en ce qu'**à chaque fois deux branches latérales (15, 16 ; 17, 18) de prothèse vasculaire sont prévues dans la première et la deuxième partie longitudinale centrale (24, 25) de coque de prothèse vasculaire, les deux branches latérales (15, 16) de prothèse vasculaire de la première partie longitudinale centrale (24) de coque de prothèse vasculaire étant disposées, par rapport à la périphérie de la coque de prothèse vasculaire (12), de manière décalée par rapport aux deux branches latérales (17, 18) de prothèse vasculaire de la deuxième partie longitudinale centrale (25) de coque de prothèse vasculaire.

7. Prothèse vasculaire auto-expansible (10) selon l'une quelconque des revendications 2 à 6, **caractérisée en ce que** les deux branches latérales (15, 16) de prothèse vasculaire de la première partie longitudinale centrale (24) de coque de prothèse vasculaire sont disposées, par rapport à un axe (A) qui s'étend perpendiculairement à l'axe longitudinal (L) s'étendant à travers la première et la deuxième partie (20, 22) de coque de prothèse vasculaire disposées concentriquement, dans la partie périphérique se rétrécissant (24a) à chaque fois à droite et à gauche de l'axe (A) suivant un angle respectif compris entre environ 30° et 60°, de préférence d'environ 45°.

8. Prothèse vasculaire auto-expansible (10) selon l'une quelconque des revendications 3 à 7, **caractérisée en ce que** les deux branches latérales (17, 18) de prothèse vasculaire de la deuxième partie longitudinale centrale (25) de coque de prothèse vasculaire sont disposées, par rapport à un axe (A) qui s'étend perpendiculairement à l'axe longitudinal (L) s'étendant à travers la première et la deuxième partie (20, 22) de coque de prothèse vasculaire disposées concentriquement, dans la partie périphérique se rétrécissant (25a) à chaque fois à droite et à gauche de l'axe (A) suivant un angle respectif compris entre environ 120° et 150°, de préférence d'environ 135°.

9. Prothèse vasculaire auto-expansible (10) selon l'une quelconque des revendications précédentes, **caractérisée en ce que** la coque de prothèse vasculaire (12) et/ou les branches latérales (15, 16, 17, 18) de prothèse vasculaire présentent des anneaux de stent (32, 33, 34, 35) s'étendant sur la circonférence à chaque fois en forme de méandres, disposés à distance les uns derrière les autres dans la direction longitudinale, lesquels anneaux de stent (32, 33, 34, 35) sont connectés les uns aux autres seulement par le biais d'un matériau de prothèse (30).

10. Prothèse vasculaire auto-expansible (10) selon l'une quelconque des revendications précédentes, **caractérisée en ce qu'**au moins dans la région des ouvertures (13, 14) et/ou des extrémités libres (15b, 17b) des branches latérales de prothèse vasculaire et/ou dans la région du branchement (15a, 15b) des branches latérales de prothèse vasculaire partant de la coque de prothèse vasculaire sont prévus des marqueurs radio-opaques (50, 51, 52).

11. Prothèse vasculaire auto-expansible (10) selon l'une quelconque des revendications précédentes, **caractérisée en ce que** les branches latérales (15, 16, 17, 18) de prothèse vasculaire partant de la coque de prothèse vasculaire (12) sont connectées à la coque de prothèse vasculaire (12) par le biais d'ouvertures ovales (40, 42) dans la partie périphérique se rétrécissant (24a, 25a) de la première et/ou de la deuxième partie longitudinale (24, 25) de coque de prothèse vasculaire.

12. Prothèse vasculaire auto-expansible (10) selon l'une quelconque des revendications précédentes, **caractérisée en ce que** la coque de prothèse vasculaire (12) est réalisée pour le positionnement dans l'aorte thoracique-abdominale et les branches latérales (15, 16, 17, 18) de prothèse vasculaire sont réalisées pour l'alimentation des artères viscérales.

13. Prothèse vasculaire auto-expansible (10) selon l'une quelconque des revendications précédentes, **caractérisée en ce qu'**elle comprend en outre au moins une première greffe de stent, avec une première et une deuxième ouverture de greffe de stent, ainsi qu'avec une lumière s'étendant entre les ouvertures de greffe de stent, la première ouverture de greffe de stent étant réalisée et dimensionnée de manière à pouvoir être introduite en partie dans la deuxième ouverture (14) de la prothèse vasculaire (10).

14. Prothèse vasculaire auto-expansible (10) selon l'une quelconque des revendications précédentes, **caractérisée en ce qu'**elle comprend en outre au moins une deuxième prothèse vasculaire, avec une première et une deuxième ouverture de prothèse vasculaire, ainsi qu'avec une lumière s'étendant entre les ouvertures de prothèse vasculaire, la première ouverture de prothèse vasculaire étant réalisée pour être introduite en partie dans les branches latérales (15, 16, 17, 18) de prothèse vasculaire en vue de l'alimentation des vaisseaux latéraux partant d'un vaisseau principal.

15. Prothèse vasculaire auto-expansible (10) selon la revendication 14, **caractérisée en ce que** la deuxième prothèse vasculaire est dotée d'un stent, ou n'est pas dotée d'un stent.
